# EUROPEAN PATENT SPECIFICATION

(11) **EP 1 677 827 B1**
(45) Date of publication and mention of the grant of the patent: **10.12.2008**
(21) Application number: 04810032.5
(22) Date of filing: 27.10.2004
(51) Int. Cl.: A61K 45/00

(54) **COMBINATIONS FOR HCV TREATMENT**
KOMBINATIONEN FÜR DIE HCV-BEHANDLUNG
INHIBITEURS COMBIN S DESTIN S AU TRAITEMENT DU VHC

(30) Priority: 27.10.2003 US 514853 P
(43) Date of publication of application: 12.07.2006
(60) Divisional application: 08102792.2
(73) Proprietor: VERTEX PHARMACEUTICALS INCORPORATED, Cambridge, MA 02139 (US)
(72) Inventor: TUNG, Roger D., San Diego, California 92130 (US); CHANDORKAR, Gurudatt, Malden, Massachusetts 02148 (US); PERNI, Robert B., Marlborough, Massachusetts 01752 (US)
(74) Representative: Cohausz & Florack
(86) International application number: PCT/US2004/035549
(87) International publication number: WO 2005/042020

(56) References cited:
- WO-A-02/100851

## Description

### TECHNICAL FIELD OF THE INVENTION

The present invention relates to combinations of a Hepatitis C virus NS3/4A protease inhibitor and a cytochrome P450 monooxygenase inhibitor. The combinations interfere with the life cycle of the hepatitis C virus and are therefore useful as antiviral therapies. As such, the combination may be used for treating or preventing Hepatitis C infections in patients. The invention also relates to compositions, kits, and pharmaceutical packs comprising the combinations. The invention also relates to processes for preparing these combinations, compositions, kits, and packs.

### BACKGROUND OF THE INVENTION

Infection by hepatitis C virus ("HCV") is a compelling human medical problem. HCV is recognized as the causative agent for most cases of non-A, non-B hepatitis, with an estimated human sero-prevalence of 3% globally [A. Alberti et al., "Natural History of Hepatitis C," J. Hepatology, 31., (Suppl. 1), pp. 17-24 (1999)]. Nearly four million individuals may be infected in the United States alone [M.J. Alter et al., "The Epidemiology of Viral Hepatitis in the United States, Gastroenterol. Clin. North Am., 23, pp. 437-455 (1994); M. J. Alter "Hepatitis C Virus Infection in the United States," J. Hepatology, 31., (Suppl. 1), pp. 88-91 (1999)].

Upon first exposure to HCV only about 20% of infected individuals develop acute clinical hepatitis while others appear to resolve the infection spontaneously. In almost 70% of instances, however, the virus establishes a chronic infection that persists for decades [S. Iwarson, "The Natural Course of Chronic Hepatitis," FEMS Microbiology Reviews, 14, pp. 201-204 (1994); D. Lavanchy, "Global Surveillance and Control of Hepatitis C," J. Viral Hepatitis, 6, pp. 35-47 (1999)]. This usually results in recurrent and progressively worsening liver inflammation, which often leads to more severe disease states such as cirrhosis and hepatocellular carcinoma [M.C. Kew, "Hepatitis C and Hepatocellular Carcinoma", FEMS Microbiology Reviews, 14, pp. 211-220 (1994); I. Saito et. al., "Hepatitis C Virus Infection is Associated with the Development of Hepatocellular Carcinoma," Proc. Natl. Acad. Sci. USA, 87, pp. 6547-6549 (1990)]. Unfortunately, there are no broadly effective treatments for the debilitating progression of chronic HCV.

The HCV genome encodes a polyprotein of 3010-3033 amino acids [Q.L. Choo, et. al., "Genetic Organization and Diversity of the Hepatitis C Virus." Proc. Natl. Acad. Sci. USA, 88, pp. 2451-2455 (1991); N. Kato et al., "Molecular Cloning of the Human Hepatitis C Virus Genome From Japanese Patients with Non-A, Non-B Hepatitis," Proc. Natl. Acad. Sci. USA, 87, pp. 9524-9528 (1990); A. Takamizawa et. al., "Structure and Organization of the Hepatitis C Virus Genome Isolated From Human Carriers," J. Virol., 65, pp. 1105-1113 (1991)]. The HCV nonstructural (NS) proteins are presumed to provide the essential catalytic machinery for viral replication. The NS proteins are derived by proteolytic cleavage of the polyprotein [R. Bartenschlager et. al., "Nonstructural Protein 3 of the Hepatitis C Virus Encodes a Serine-Type Proteinase Required for Cleavage at the NS3/4 and NS4/5 Junctions," J. Virol., 67, pp. 3835-3844 (1993); A. Grakoui et. al., "Characterization of the Hepatitis C Virus-Encoded Serine Proteinase: Determination of Proteinase-Dependent Polyprotein Cleavage Sites," J. Virol., 67, pp. 2832-2843 (1993); A. Grakoui et. al., "Expression and Identification of Hepatitis C Virus Polyprotein Cleavage Products," J. Virol., 67, pp. 1385-1395 (1993); L. Tomei et. al., "NS3 is a serine protease required for processing of hepatitis C virus polyprotein", J. Virol., 67, pp. 4017-4026 (1993)].

The HCV NS protein 3 (NS3) contains a serine protease activity that helps process the majority of the viral enzymes, and is thus considered essential for viral replication and infectivity. It is known that mutations in the yellow fever virus NS3 protease decreases viral infectivity [Chambers, T.J. et. al., "Evidence that the N-terminal Domain of Nonstructural Protein NS3 From Yellow Fever Virus is a Serine Protease Responsible for Site-Specific Cleavages in the Viral Polyprotein", Proc. Natl. Acad. Sci. USA, 87, pp. 8898-8902 (1990)]. The first 181 amino acids of NS3 (residues 1027-1207 of the viral polyprotein) have been shown to contain the serine protease domain of NS3 that processes all four downstream sites of the HCV polyprotein [C. Lin et al., "Hepatitis C Virus NS3 Serine Proteinase: Trans-Cleavage Requirements and Processing Kinetics", J. Virol., 68, pp. 8147-8157 (1994)].

There are not currently any satisfactory anti-HCV agents or treatments. Until recently, the only established therapy for HCV disease was interferon treatment. However, interferons have significant side effects [M. A. Wlaker et al., "Hepatitis C Virus: An Overview of Current Approaches and Progress," DDT, 4, pp. 518-29 (1999); D. Moradpour et al., "Current and Evolving Therapies for Hepatitis C," Eur. J. Gastroenterol. Hepatol., 11, pp. 1199-1202 (1999); H. L. A. Janssen et al. "Suicide Associated with Alfa-Interferon Therapy for Chronic Viral Hepatitis," J. Hepatol., 21, pp. 241-243 (1994); P.F. Renault et al., "Side Effects of Alpha Interferon," Seminars in Liver Disease, 9, pp. 273-277. (1989)] and induce long term remission in only a fraction (- 25%) of cases [O. Weiland, "Interferon Therapy in Chronic Hepatitis C Virus Infection" , FEMS Microbiol. Rev., 14, pp. 279-288 (1994)]. Recent introductions of the pegylated forms of interferon (PEG-INTRON^{®} and PEGASYS^{®}) and the combination therapy of ribavirin and pegylated interferon (REBETROL^{®}) have resulted in only modest improvements in remission rates and only partial reductions in side effects. Moreover, the prospects for effective anti-HCV vaccines remain uncertain.

The HCV NS3 serine protease and its associated cofactor, NS4A, helps process all of the viral enzymes, and is thus considered essential for viral replication. This processing appears to be analogous to that carried out by the human immunodeficiency virus aspartyl protease, which is also involved in viral enzyme processing HIV protease inhibitors, which inhibit viral protein processing are potent antiviral agents in man, indicating that interrupting this stage of the viral life cycle results in therapeutically active agents. Consequently it is an attractive target for drug discovery.

It is known that some drugs are metabolized by cytochrome p450 enzymes. Such metabolism typically results in the drug having unfavorable pharmacokinetic characteristics (e.g., decreased blood levels, decreased half-life). In contrast, inhibition of drug metabolism can lead to improvements in the pharmacokinetic profile of the drug. This approach has led to reports of methods for improving the pharmacokinetics of certain drugs [see, e.g., US Patent 6,037,157; D.E. Kempf et al. Antimicrob. Agents Chemother., 41, pp. 654-660 (1997)]. However, there have been no reports of methods for improving the pharmacokinetics of Hepatitis C virus NS3/4A protease inhibitors.

Thus, there is a need for compositions and therapeutic combinations for improving the pharmacokinetics of Hepatitis C NS3/4A virus protease inhibitors. Such compositions, combinations, and methods would be useful in anti-HCV therapies.

### SUMMARY OF THE INVENTION

The present invention relates to combinations of a Hepatitis C virus NS3/4A protease inhibitor VX-950 and a cytochrome P450 monooxygenase inhibitor ritonavir.

The invention provides compositions, kits, and pharmaceutical packs comprising the Hepatitis C virus NS3/4A protease inhibitor the cytochrome P450 monooxygenase inhibitor. The invention also provides processes for preparing these combinations, kits, and packs.

### DETAILED DESCRIPTION OF THE INVENTION

This invention provides methods for improving the pharmacokinetics of the Hepatitis C NS3/4A protease inhibitors. The advantages of improving the pharmacokinetics of drugs are recognized in the art (US 2004/0091527; US 2004/0152625; US 2004/0091527). Such improvement may lead to increased blood levels of the drug. More importantly for HCV therapies, the improvement may lead to increased concentrations of the protease inhibitor in the liver.

Applicants have demonstrated that Hepatitis C NS3/4A protease inhibitors are metabolized by the cytochrome P450 enzymes, particularly the 3A4 isozyme. Applicants have also demonstrated that that this metabolism is decreased in the presence of a cytochrome P450 inhibitor. By combining a NS3/4A protease inhibitor and a CYP inhibitor, this invention provides for decreased metabolism of protease inhibitors. The pharmacokinetics of the protease inhibitor are thereby improved.

Accordingly, this invention provides therapeutic combinations of a cytochrome P450 monooxygenase inhibitor ("CYP inhibitor") and a Hepatitis C NS3/4A protease inhibitor.

One embodiment of this invention provides a method for increasing the bioavailability of a Hepatitis C virus NS3/4A protease inhibitor in a patient comprising administering to the patient a combination of Hepatitis C NS3/4A virus protease inhibitor and a cytochrome P450 monooxygenase inhibitor.

Another embodiment of this invention provides a method for treating a patient infected with a Hepatitis C virus comprising administering to the patient a) a Hepatitis C virus NS3/4A protease inhibitor; and b) a cytochrome P450 monooxygenase inhibitor.

The cytochrome P450 monooxygenase inhibitor used in this invention is expected to inhibit metabolism of the NS3/4A protease inhibitor compound. Therefore, the cytochrome P450 monooxygenase inhibitor would be in an amount effective to inhibit metabolism of the protease inhibitor. Accordingly, the CYP inhibitor is administered in an amount such that the bioavailiablity of the protease inhibitor is increased in comparison to the bioavailability in the absence of the CYP inhibitor.

In these embodiments, the inhibitor is preferably administered in therapeutically effective amounts. The compound used in this invention is expected to inhibit HCV by inhibiting NS3/4A protease. As such, a combination of the CYP inhibitor and the protease inhibitor are preferably co-administered in an amount sufficient to have antiviral activity.

It should be understood that as this invention involves a combination of compounds, the specific amounts of each compound may be dependent on the specific amounts of each other compound in the combination. For example, the administration of CYP inhibitor and a protease inhibitor in a method of this invention, leads to improved pharmacokinetics of the protease inhibitor as compared to the pharmacokinetics of the protease inhibitor administered in the absence of CYP inhibitor. Therefore, a lower amount of protease inhibitor would give an equivalent effect in vivo in the presence of a CYP inhibitor than in the absence of the CYP inhibitor.

The amount of ritonavir administered to a patient is preferably sufficient to improve the pharmacokinetics of the protease inhibitor VX-950 as compared to the pharmacokinetics of the protease inhibitor VX-950 in the absence of ritonavir. Preferably, the amount of ritonavir administered is sufficient to increase the blood levels of the protease inhibitor VX-950 or to increase the liver concentrations of the protease inhibitor VX-950. Advantageously, a lower dose of protease inhibitor VX-950 is therefore used (relative to administration of the protease inhibitor VX-950 alone).

In addition to treating patients infected with Hepatitis C, the invention may be used to prevent a patient from becoming infected with Hepatitis C. Accordingly, one embodiment of this invention provides a method for preventing a Hepatitis C virus infection in a patient comprising administering to the patient a) a Hepatitis C virus NS3/4A protease inhibitor; and b) a cytochrome P450 monooxygenase inhibitor.

As would be realized by skilled practitioners, the invention is being used to treat a patient prophylactically, and that patient becomes infected with Hepatitis C virus, the method may then treat the infection. Therefore, one embodiment of this invention provides a Hepatitis C virus NS3/4A protease inhibitor and a cytochrome P450 monooxygenase inhibitor wherein the combination of inhibitors are in therapeutically effective amounts for treating or preventing a Hepatitis C infection in a patient.

VX-950 is a competitive, reversible peptidomimetic NS3/4A protease inhibitor with a steady state binding constant (ki*) of 3nM [WO 02/018369].

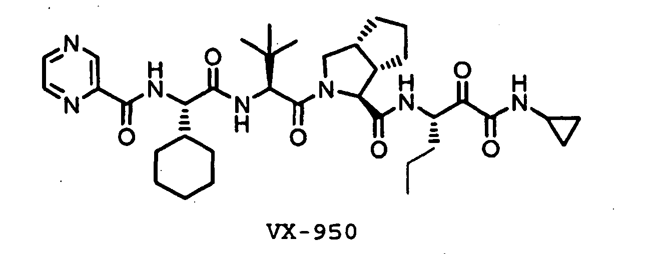

Accordingly, the NS3/4A protease inhibitor for use in the methods, processes, combinations, compositions, packs, and kits of present invention is VX-950.

Preferred compounds for use in connection with this invention are those wherein the compound is sufficiently stable to allow manufacture and administration to a mammal by methods known in the art. Typically, such compounds are stable at a temperature of 40°C or less, in the absence of moisture or other chemically reactive condition, for at least a week.

VX-950 may contain one or more asymmetric carbon atoms and thus may occur as racemates and racemic mixtures, single enantiomers, diastereomeric mixtures and individual diastereomers. All such isomeric forms of these compounds are expressly included in the present invention. Each stereogenic carbon may be of the R or S configuration. The D- and L-isomers at the N-propyl side chain of VX-950 are expressly included within this invention.

The CYP inhibitor is to, ritonavir (WO 94/14436),

Methods for measuring the ability of a compound to inhibit cytochrome P50 monooxygenase activity are known (see, US 6,037,157 and Yun, et al. Drug Metabolism & Disposition, vol. 21, pp. 403-407 (1993)). For example, A compound to be evaluated may be incubated with 0.1, 0.5, and 1.0 mg protein/ml, or other appropriate concentration of human hepatic microsomes (e.g., commercially available, pooled characterized hepatic microsomes) for 0, 5, 10, 20, and 30 minutes, or other appropriate times, in the presence of an NADPH-generating system. Control incubations may be performed in the absence of hepatic microsomes for 0 and 30 minutes (triplicate). The samples may be analyzed for the presence of the compound. Incubation conditions that produce a linear rate of compound metabolism will be used a guide for further studies.

Typical experiments would determine the kinetics of the compound's metabolism (Kₘ and Vₘₐₓ). The rate of disappearance of compound may be determined and the data analyzed according to Michaelis-Menten kinetics by using Lineweaver-Burk, Eadie-Hofstee, or nonlinear regression analysis.

Inhibition of metabolism experiments may then be performed. For example, a compound (one concentration, s Kₘ) may be incubated with pooled human hepatic microsomes in the absence or presence of a CYP inhibitor (such as ritonavir) under the conditions determined above. As would be recognized, control incubations should contain the same concentration of organic solvent as the incubations with the CYP inhibitor. The concentrations of the compound in the samples may be quantitated, and the rate of disappearance of parent compound may be determined, with rates being expressed as a percentage of control activity.

Methods for evaluating the influence of coadministration of the NS3/4A protease inhibitor and the CYP inhibitor in a subject are also known (US2004/0028755). Any such methods could be used in connection with this invention to determine the pharmacokinetic impact of a combination. Subjects that would benefit from treatment according to this invention could then be selected. Specifically, subjects that metabolize NS3/4A protease inhibitors could be chosen for treatment according to this invention. Subjects that metabolize NS3/4A protease inhibitors extensively or at least to a greater extent than other subjects would be preferred subjects for treatment according to this invention.

A CYP inhibitor employed in this invention may be an inhibitor of only one isozyme or more than one isozyme. If the CYP inhibitor inhibits more isozyme, the inhibitor may nevertheless inhibit one isozyme more selectively than another isozyme. Any such CYP inhibitors may be used in a method of this invention.

As would be appreciated, CYP3A4 activity is broadly observed in humans. Accordingly, embodiments of this invention involving inhibition of isozyme 3A4 would be expected to be applicable to a broad range of patients.

The methods herein involve administration of combinations of a Hepatitis C virus NS3/4A protease inhibitor and a cytochrome P450 monooxygenase inhibitor. Such administration may be referred to as co-administration. Co-administration includes administering each inhibitor in the same dosage form or in different dosage forms. When administered in different dosage forms, the inhibitors may be administered at different times and in any order.

Accordingly, this invention provides methods wherein the CYP inhibitor is administered together with the Hepatitis C virus NS3/4A protease inhibitor in the same dosage form or in separate dosage forms.

If the CYP inhibitor and protease inhibitor are administered in separate dosage forms, each inhibitor may be administered about simultaneously. Alternatively, the CYP inhibitor may be administered in any time period around administration of the protease inhibitor. That is, the CYP inhibitor may be administered prior to, together with, or following the NS3/4A protease inhibitor. The time period of administration should be such that the CYP inhibitor affects the metabolism of the protease inhibitor. For example, if the protease inhibitor is administered first, the CYP inhibitor should be administered before the protease inhibitor is metabolized and/or excreted (e.g., within the half-life of the protease inhibitor).

The invention may also involve administration of another component comprising an additional agent selected from an immunomodulatory agent; an antiviral agent; an inhibitor of HCV protease; an inhibitor of another target in the HCV life cycle; a cytochrome P-450 inhibitor; or combinations thereof.

Accordingly, this invention provides a method comprising administering a NS3/4A protease inhibitor, a CYP inhibitor, and another anti-viral agent, preferably an anti-HCV agent. Such anti-viral agents include, but are not limited to, immunomodulatory agents, such as α-, β-, and γ-interferons, pegylated derivatized interferon-α compounds, and thymosin; other anti-viral agents, such as ribavirin, amantadine, and telbivudine; other inhibitors of hepatitis C proteases (NS2-NS3 inhibitors and NS3-NS4A inhibitors); inhibitors of other targets in the HCV life cycle, including helicase, polymerase, and metalloprotease inhibitors; inhibitors of internal ribosome entry; broad-spectrum viral inhibitors, such as IMPDH inhibitors (e.g., compounds of United States Patent 5,807,876, 6,498,178, 6,344,465, 6,054,472, WO 97/40028, WO 98/40381, WO 00/56331, and mycophenolic acid and derivatives thereof, and including, but not limited to VX-497, VX-148, and/or VX-944); or combinations of any of the above.

Other agents (e.g., non-immunomodulatory or immunomodulatory compounds) may be used in combination with a compound of this invention include, but are not limited to, those specified in WO 02/18369, which is incorporated herein by reference (see, e.g., page 273, lines 9-22 and page 274, line 4 to page 276, line 11).

Still other agents include, but are not limited to, PEG-INTRON^{®} (peginteferon alfa-2b, available from Schering Corporation, Kenilworth, NJ); INTRON-A^{®}, (interferon alfa-2b available from Schering Corporation, Kenilworth, NJ); ribavirin (1-beta-D-ribofuranosyl-1H-1,2,4-triazole-3-carboxamide, available from ICN Pharmaceuticals, Inc., Costa Mesa, CA; described in the Merck Index, entry 8365, Twelfth Edition); REBETROL^{®} (Schering Corporation, Kenilworth, NJ), COPEGASUS^{®} (Hoffmann-La Roche, Nutley, NJ); PEGASYS^{®} (peginterferon alfa-2a available Hoffmann-La Roche, Nutley, NJ); ROFERON^{®} (recombinant interferon alfa-2a available from Hoffmann-La Roche, Nutley, NJ); BEREFOR^{®} (interferon alfa 2 available from Boehringer Ingelheim Pharmaceutical, Inc., Ridgefield, CT); SUMIFERON^{®} (a purified blend of natural alpha interferons such as Sumiferon available from Sumitomo, Japan); WELLFERON^{®} (interferon alpha n1 available from Glaxo Wellcome Ltd., Great Britain); ALFERON^{®} (a mixture of natural alpha interferons made by Interferon Sciences, and available from Purdue Frederick Co., CT); α-interferon; natural alpha interferon 2a; natural alpha interferon 2b; pegylated alpha interferon 2a or 2b; consensus alpha interferon (Amgen, Inc., Newbury Park, CA); VIRAFERON^{®}; INFERGEN^{®}; REBETRON^{®} (Schering Plough, Inteferon-alpha 2B + Ribavirin); pegylated interferon alpha (Reddy, K.R. et al. "Efficacy and Safety of Pegylated (40-kd) Interferon alpha-2a Compared with Interferon alpha-2a in Noncirrhotic Patients with Chronic Hepatitis C (Hepatology, 33, pp. 433-438 (2001); consensus interferon (Kao, J.H., et al., "Efficacy of Consensus Interferon in the Treatment of Chronic Hepatitis" J. Gastroenterol. Hepatol. 15, pp. 1418-1423 (2000); lymphoblastoid or "natural" interferon; interferon tau (Clayette, P. et al., "IFN-tau, A New Interferon Type I with Antiretroviral activity" Pathol. Biol. (Paris) 47, pp. 553-559 (1999); interleukin 2 (Davis, G.L. et al., "Future Options for the Management of Hepatitis C." Seminars in Liver Disease, 19, pp. 103-112 (1999); Interleukin 6 (Davis et al. "Future Options for the Management of Hepatitis C." Seminars in Liver Disease 19, pp. 103-112 (1999); interleukin 12 (Davis, G.L. et al., "Future Options for the Management of Hepatitis C." Seminars in Liver Disease, 19, pp. 103-112 (1999); and compounds that enhance the development of type 1 helper T cell response (Davis et al., "Future Options for the Management of Hepatitis C." Seminars in Liver Disease, 19, pp. 103-112 (1999)). Also included are compounds that stimulate the synthesis of interferon in cells (Tazulakhova, E.B. et al., "Russian Experience in Screening, analysis, and Clinical Application of Novel Interferon Inducers" J. Interferon Cytokine Res., 21 pp. 65-73) including, but are not limited to, double stranded RNA, alone or in combination with tobramycin, and Imiquimod (3M Pharmaceuticals; Sauder, D.N. "Immunomodulatory and Pharmacologic Properties of Imiquimod" J. Am. Acad. Dermatol., 43 pp. S6-11 (2000).

As is recognized by skilled practitioners, a protease inhibitor and a CYP inhibitor would be preferably administered orally. Interferon is not typically administered orally. Nevertheless, nothing herein limits the methods or combinations of this invention to any specific dosage forms or regime. Thus, each component of a combination according to this invention may be administered separately, together, or in any combination thereof. As recognized by skilled practitioners, dosages of interferon are typically measured in IU (e.g., about 4 million IU to about 12 million IU).

If an additional agent is selected from another CYP inhibitor, the method would, therefore, employ two or more CYP inhibitors. Each component may be administered in one or more dosage forms. Each dosage form may be administered to the patient in any order.

The NS3/4A protease inhibitor, the CYP inhibitor, and any additional agent may be formulated in separate dosage forms. Alternatively, to decrease the number of dosage forms administered to a patient, the NS3/4A protease inhibitor, the CYP inhibitor, and any additional agent may be formulated together in any combination. For example, the NS3/4A protease inhibitor may be formulated in one dosage form and the CYP inhibitor and the additional agent may be formulated together in another dosage form. Any separate dosage forms may be administered at the same time or different times. It should be understood that CYP inhibitor would be administered within a time period such that the CYP inhibitor would decrease the metabolism of the NS3/4A protease inhibitor (or the additional agent or agents).

Accordingly, another embodiment of this invention provides a composition comprising a NS3/4A protease inhibitor, or a pharmaceutically acceptable salt thereof, and a CYP inhibitor, or a pharmaceutically acceptable salt thereof. According to a preferred embodiment, the NS3/4A protease inhibitor is present in an amount effective to decrease the viral load in a sample or in a patient, wherein said virus encodes a NS3/4A serine protease necessary for the viral life cycle, and a pharmaceutically acceptable carrier. Alternatively, a composition of this invention comprises an additional agent as described herein. Each component may be present in individual compositions, combination compositions, or in a single composition.

If pharmaceutically acceptable salts of compounds are utilized in these compositions, those salts are preferably derived from inorganic or organic acids and bases. Included among such acid salts are the following: acetate, adipate, alginate, aspartate, benzoate, benzene sulfonate, bisulfate, butyrate, citrate, camphorate, camphor sulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate and undecanoate. Base salts include ammonium salts, alkali metal salts, such as sodium and potassium salts, alkaline earth metal salts, such as calcium and magnesium salts, salts with organic bases, such as dicyclohexylamine salts, N-methyl-D-glucamine, and salts with amino acids such as arginine, lysine, and so forth.

Also, the basic nitrogen-containing groups may be quaternized with such agents as lower alkyl halides, such as methyl, ethyl, propyl, and butyl chloride, bromides and iodides; dialkyl sulfates, such as dimethyl, diethyl, dibutyl and diamyl sulfates, long chain halides such as decyl, lauryl, myristyl and stearyl chlorides, bromides and iodides, aralkyl halides, such as benzyl and phenethyl bromides and others. Water or oil-soluble or dispersible products are thereby obtained.

The compounds utilized in the compositions and methods of this invention may also be modified by appending appropriate functionalities to enhance selective biological properties. Such modifications are known in the art and include those which increase biological penetration into a given biological system (e.g., blood, lymphatic system, central nervous system), increase oral availability, increase solubility to allow administration by injection, alter metabolism and alter rate of excretion.

Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

According to a preferred embodiment, the compositions of this invention are formulated for pharmaceutical administration to a mammal, particularly a human being.

Such pharmaceutical compositions of the present invention (as well as compositions for use in methods, combinations, kits, and packs of this inventions) may be administered orally, parenterally, sublingually, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally or intravenously. More preferably, the compositions are administered orally.

Sterile injectable forms of the compositions of and according to this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

In compositions of this invention, and according to this invention (i.e., compositions used in methods, kits, combinations, or packs of this invention), both the NS3/4A protease inhibitor, the CYP inhibitor, and any optional additional agent should be present at dosage levels of between about 10 to 100%, and more preferably between about 10 to 80% of the dosage normally administered in a monotherapy regimen.

The pharmaceutical compositions of this invention may be orally administered in any orally acceptable dosage form including, but not limited to, capsules, tablets, pills, powders, granules, aqueous suspensions or solutions. In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added. Acceptable liquid dosage forms include emulsions, solutions, suspensions, syrups, and elixirs.

Alternatively, the pharmaceutical compositions of this invention may be administered in the form of suppositories for rectal administration. These may be prepared by mixing the agent with a suitable non-irritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract may be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions may be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions of, and according to, this invention may also be administered by nasal aerosol or inhalation. Such compositions are prepared according to techniques well known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

As is recognized in the art, pharmaceutical compositions may also be administered in the form of liposomes.

Preferred are pharmaceutical compositions of, and according to, this invention formulated for oral administration.

Dosage levels of between about 0.01 and about 100 mg/kg body weight per day, preferably between about 0.5 and about 75 mg/kg body weight per day of the NS3/4A protease inhibitor are useful for the prevention and treatment of HCV mediated disease. For the CYP inhibitor, the dosage levels of between about 0.001 to about 200 mg/kg body weight per day, would be typical. More typical would be dosage levels of between about 0.1 to about 50 mg/kg or about 1.1 to about 25 mg/kg per day. Typically, the pharmaceutical compositions of, and according to, this invention will be administered from about 1 to about 5 times per day or alternatively, as a continuous infusion. Such administration can be used as a chronic or acute therapy. The amount of active ingredient that may be combined with the carrier materials to produce a single dosage form will vary depending upon the host treated and the particular mode of administration.
A typical preparation will contain from about 5% to about 95% active compound (w/w). Preferably, such preparations contain from about 20% to about 80% active compound.

Upon improvement of a patient's condition, a maintenance dose of a compound, composition or combination of this invention may be administered, if necessary. Subsequently, the dosage or frequency of administration, or both, may be reduced, as a function of the symptoms, to a level at which the improved condition is retained when the symptoms have been alleviated to the desired level, treatment should cease. Patients may, however, require intermittent treatment on a long-term basis upon any recurrence of disease symptoms.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredients will also depend upon the particular described compound and the presence or absence and the nature of the additional anti-viral agent in the composition.

For preferred dosage forms of ritonavir, see United States Patent 6,037, 157, and the documents cited therein: United States Patent 5,484,801, United States Application 08/402,690, and International Applications WO 95/07696 and WO 95/09614).

According to another embodiment, the invention provides a method for treating a patient infected with a virus characterized by a virally encoded NS3/4A serine protease that is necessary for the life cycle of the virus by administering to said patient a pharmaceutically acceptable composition of this invention. Preferably, the methods of this invention are used to treat a patient suffering from a HCV infection. Such treatment may completely eradicate the viral infection or reduce the severity thereof. More preferably, the patient is a human being.

In yet another embodiment the present invention provides a method of pre-treating a biological substance intended for administration to a patient comprising the step of contacting said biological substance with a pharmaceutically acceptable composition comprising a compound of this invention. Such biological substances include, but are not limited to, blood and components thereof such as plasma, platelets, subpopulations of blood cells and the like; organs such as kidney, liver, heart, lung, etc; sperm and ova; bone marrow and components thereof, and other fluids to be infused into a patient such as saline, dextrose, etc.

This invention also provides a process for preparing a composition comprising a Hepatitis C virus NS3/4A protease inhibitor and a cytochrome P450 monooxygenase inhibitor, comprising the step of combining the Hepatitis C virus NS3/4A protease inhibitor and the cytochrome P450 monooxygenase inhibitor. An alternative embodiment of this invention provides a process wherein the composition comprises one or more additional agent.as described herein.

This invention also provides a therapeutic combination comprising a Hepatitis C virus NS3/4A protease inhibitor and a cytochrome P450 monooxygenase inhibitor. In an alternative embodiment of this invention, the therapeutic combination further comprises one or more of additional agent as described herein.

Pharmaceutical compositions may also be prescribed to the patient in "patient packs" containing the whole course of treatment in a single package, usually a blister pack. Patient packs have an advantage over traditional prescriptions, where a pharmacists divides a patients supply of a pharmaceutical from a bulk supply, in that the patient always has access to the package insert contained in the patient pack, normally missing in traditional prescriptions. The inclusion of a package insert has been shown to improve patient compliance with the physician's instructions.

It will be understood that the administration of the combination of the invention by means of a single patient pack, or patient packs of each formulation, containing within a package insert instructing the patient to the correct use of the invention is a desirable additional feature of this invention.

According to a further aspect of the invention is a pack comprising at least a NS3/4A protease inhibitor and a CYP inhibitor of the invention and an information insert containing directions on the use of the combination of the invention. In an alternative embodiment of this invention, the pharmaceutical pack further comprises one or more of additional agent as described herein. The additional agent or agents may be provided in the same pack or in separate packs.

Another aspect of this involves a packaged kit for a patient to use in the treatment of HCV infection or in the prevention of HCV infection, comprising: a single or a plurality of pharmaceutical formulation of each pharmaceutical component; a container housing the pharmaceutical formulation(s) during storage and prior to administration; and instructions for carrying out drug administration in a manner effective to treat or prevent HCV infection.

Accordingly, this invention provides kits for the simultaneous or sequential administration of a NS3/4A protease inhibitor and a CYP inhibitor (and optionally an additional agent) or derivatives thereof are prepared in a conventional manner. Typically, such a kit will comprise, e.g. a composition of each inhibitor and optionally the additional agent(s) in a pharmaceutically acceptable carrier (and in one or in a plurality of pharmaceutical formulations) and written instructions for the simultaneous or sequential administration.

In another embodiment, a packaged kit is provided that contains one or more dosage forms for self administration; a container means, preferably sealed, for housing the dosage forms during storage and prior to use; and instructions for a patient to carry out drug administration. The instructions will typically be written instructions on a package insert, a label, and/or on other components of the kit, and the dosage form or forms are as described herein. Each dosage form may be individually housed, as in a sheet of a metal foil-plastic laminate with each dosage form isolated from the others in individual cells or bubbles, or the dosage forms may be housed in a single container, as in a plastic bottle. The present kits will also typically include means for packaging the individual kit components, i.e., the dosage forms, the container means, and the written instructions for use. Such packaging means may take the form of a cardboard or paper box, a plastic or foil pouch, etc.

Although certain exemplary embodiments are depicted and described below, it will be appreciated that compounds of this invention can be prepared according to the methods described generally above using appropriate starting materials generally available to one of ordinary skill in the art.

In order that this invention be more fully understood, the following preparative and testing examples are set forth. These examples are for the purpose of illustration only and are not to be construed as limiting the scope of the invention in any way.

### Example 1

### HCV Replicon Cell Assay Protocol

Cells containing hepatitis C virus (HCV) replicon were maintained in DMEM containing 10% fetal bovine serum (FBS), 0.25 mg per ml of G418, with appropriate supplements (media A).

On day 1, replicon cell monolayer was treated with a trypsin:EDTA mixture, removed, and then media A was diluted into a final concentration of 100,000 cells per ml wit. 10,000 cells in 100 µl were plated into each well of a 96-well tissue culture plate, and cultured overnight in a tissue culture incubator at 37°C.

On day 2, compounds (in 100% DMSO) were serially diluted into DMEM containing 2% FBS, 0.5% DMSO, with appropriate supplements (media B). The final concentration of DMSO was maintained at 0.5% throughout the dilution series.

Media on the replicon cell monolayer was removed, and then media B containing various concentrations of compounds was added. Media B without any compound was added to other wells as no compound controls.

Cells were incubated with compound or 0.5% DMSO in media B for 48 hours in a tissue culture incubator at 37°C. At the end of the 48-hour incubation, the media was removed, and the replicon cell monolayer was washed once with PBS and stored at -80°C prior to RNA extraction.

Culture plates with treated replicon cell monolayers were thawed, and a fixed amount of another RNA virus, such as Bovine Viral Diarrhea Virus (BVDV) was added to cells in each well. RNA extraction reagents (such as reagents from RNeasy kits) were added to the cells immediately to avoid degradation of RNA. Total RNA was extracted according the instruction of manufacturer with modification to improve extraction efficiency and consistency. Finally, total cellular RNA, including HCV replicon RNA, was eluted and stored at -80°C until further processing.

A Taqman real-time RT-PCR quantification assay was set up with two sets of specific primers and probe. One was for HCV and the other was for BVDV. Total RNA extractants from treated HCV replicon cells was added to the PCR reactions for quantification of both HCV and BVDV RNA in the same PCR well. Experimental failure was flagged and rejected based on the level of BVDV RNA in each well. The level of HCV RNA in each well was calculated according to a standard curve run in the same PCR plate. The percentage of inhibition or decrease of HCV RNA level due to compound treatment was calculated using the DMSO or no compound control as 0% of inhibition. The IC50 (concentration at which 50% inhibition of HCV RNA level is observed) was calculated from the titration curve of any given compound.

### Example 2

### HCV Ki Assay Protocol

### HPLC Microbore method for separation of 5AB substrate and products

Substrate:
NH₂-Glu-Asp-Val-Val-(alpha)Abu-Cys-Ser-Met-Ser-Tyr-COOH

A stock solution of 20 mM 5AB (or concentration of your choice) was made in DMSO w/ 0.2M DTT. This was stored in aliquots at -20 C.

Buffer: 50 mM HEPES, pH 7.8; 20% glycerol; 100 mM NaCl

Total assay volume was 100 µL

| | X1 (µL) | conc. in assay |
|---|---|---|
| Buffer | 86.5 | See above |
| 5 mM KK4A | 0.5 | 25 µM |
| 1 M DTT | 0.5 | 5 mM |
| DMSO or inhibitor | 2.5 | 2.5% v/v |
| 50 µM tNS3 | 0.05 | 25 nM |
| 250 µM 5AB (initiate) | 20 | 25 µM |

The buffer, KK4A, DTT, and tNS3 were combined; distributed 78 µL each into wells of 96 well plate. This was incubated at 30 C for ∼5-10 min.

2.5 µL of appropriate concentration of test compound was dissolved in DMSO (DMSO only for control) and added to each well. This was incubated at room temperature for 15 min.

Initiated reaction by addition of 20 µL of 250 µM 5AB substrate (25 µM concentration is equivalent or slightly lower than the Km for 5AB).
Incubated for 20 min at 30 C.
Terminated reaction by addition of 25 µL of 10% TFA
Transferred 120 µL aliquots to HPLC vials

Separated SMSY product from substrate and KK4A by the following method:

### Microbore separation method:

**Instrumentation: Agilent 1100**
Degasser G1322A
Binary pump G1312A
Autosampler G1313A
Column thermostated chamber G1316A
Diode array detector G1315A

### Column:

Phenomenex Jupiter; 5 micron C18; 300 angstroms; 150x2 mm; P/O 00F-4053-B0
Column thermostat: 40 C
Injection volume: 100 µL
Solvent A = HPLC grade water + 0.1% TFA
Solvent B = HPLC grade acetonitrile + 0.1% TFA

| Time (min) | %B | Flow (ml/min) | Max press. |
|---|---|---|---|
| 0 | 5 | 0.2 | 400 |
| 12 | 60 | 0.2 | 400 |
| 13 | 100 | 0.2 | 400 |
| 16 | 100 | 0.2 | 400 |
| 17 | 5 | 0.2 | 400 |

Stop time: 17 min
Post-run time: 10 min.

### Example 3

### Metabolic Stability of NS3/4A Protease Inhibitors Interaction of Ritonavir in the Metabolism of VX-950

The metabolism of VX-950 and the interaction with ritonavir was investigated using human hepatic microsomes. Initial incubations were performed in a 0.1 M phosphate buffer, pH 7.4, containing 1 mM EDTA, NADPH, 1 µM VX-950, and either 0.1, 0.5, or 1.0 mg microsomal protein/mL for various time points. Due to non-linear rates of metabolism, additional incubations were performed containing either 0.25 or 0.5 mg microsomal protein/mL at two concentrations of VX-950 (0.5 and 1 µM) for up to 30 minutes.

Due to the apparent rapid metabolism of VX-950 in human hepatic microsomes, the kinetics (Vₘₐₓ and Km) of VX-950 metabolism was determined using a protein concentration of 0.25 mg/mL and an incubation time of 2 minutes. The interaction of ritonavir on the metabolism of VX-950 was determined by incubating a single concentration of VX-950 (0.25 µM) and human hepatic microsomes (0.25 mg microsomal protein/mL) with various concentrations of ritonavir (0 to 100 µM) for 2 minutes. The addition of ritonavir at concentrations up to 3 µM produced inhibition of VX-950 metabolism. At higher concentrations of ritonavir (10 to 100 µM) an increase in VX-950 metabolism was observed.

In summary, VX-950 at concentrations used in this study is rapidly metabolized in human liver microsomes (e.g., 73% @ 2µM at 60 min., 7% @ 20µM; or 86% @ 2µM at 120 min., 21% @ 20µM). Other NS3/4A protease inhibitors exhibited similar results.

Ritonavir has been demonstrated to inhibit the metabolism of VX-950. However, following interaction with high concentrations of ritonavir, activation of VX-950 metabolism occurs. The mechanism of this increase in VX-950 metabolism in the presence of ritonavir is unclear. Without being bound by theory, this increase may be the result of simultaneous binding of both compounds in the same active site, or abolishment of CYP3A4 activity by ritonavir may result in VX-950 being metabolized by other non-inhibited CYP450 enzymes.

## Claims

1. A pharmaceutical composition comprising: (a) the Hepatitis C virus NS3/4A protease inhibitor 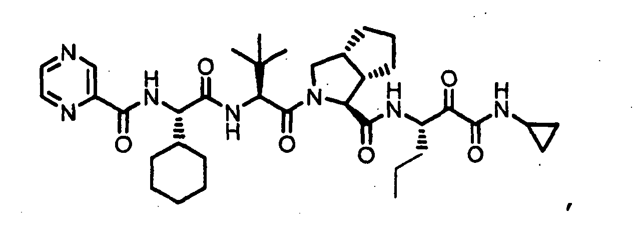 a stereoisomer thereof or a pharmaceutically acceptable salt thereof; (b) ritonavir or a pharmaceutically acceptable salt thereof; and (c) a pharmaceutically acceptable carrier.

2. The use of the Hepatitis C virus NS3/4A protease inhibitor 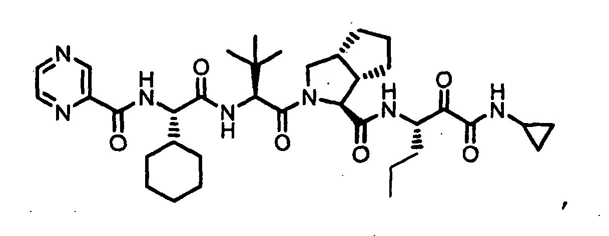 a stereoisomer thereof or a pharmaceutically acceptable salt thereof, and ritonavir or a pharmaceutically acceptable salt thereof, for the preparation of a medicament for treating or preventing a Hepatitis C virus infection.

3. The use according to claim 2, wherein the Hepatitic C virus NS3/4A protease inhibitor and ritonavir are in separate dosage forms.

4. The use according to claim 3 wherein the separate dosage forms are administered about simultaneously.

5. The use according to claim 2, wherein the Hepatitis C virus NS3/4A protease inhibitor and ritonavir are in a single dosage form.

6. The use according to any one of claims 2 to 5 wherein said use comprises administering an additional agent selected from an immunomodulatory agent; an antiviral agent; another inhibitor of HCV NS3/4A protease; an inhibitor of a target in the HCV life cycle other than NS3/4A protease; an inhibitor of internal ribosome entry, a broad-spectrum viral inhibitor; another cytochrome P-450 inhibitor; or combinations thereof.

7. The use according to claim 6, wherein said immunomodulatory agent is α-, β-, or γ-interferon or thymosin; the antiviral agent is ribavirin, amantadine, or telbivudine; or the inhibitor of another target in the HCV life cycle is an inhibitor of HCV helicase, polymerase, or metalloprotease.

## Patentansprüche

1. Pharmazeutische Zusammensetzung enthaltend:
(a) den Hepatitis-C-Virus-NS3/4A-Protease-Inhibitor 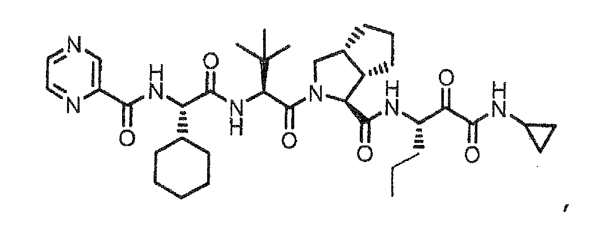 ein Stereoisomer oder ein pharmazeutisch verträgliches Salz davon,
(b) Ritonavir oder ein pharmazeutisch verträgliches Salz davon und
(c) einen pharmazeutisch verträglichen Träger.

2. Verwendung von dem Hepatitis-C-Virus-NS3/4A-Protease-Inhibitor 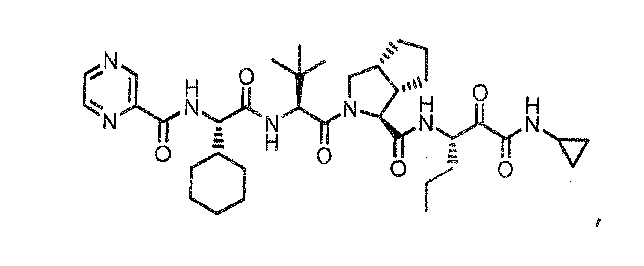 einem Stereoisomer oder einem pharmazeutisch verträglichen Salz davon und Ritonavir oder einem pharmazeutisch verträglichen Salz davon zur Herstellung eines Arzneimittels für die Behandlung oder Verhütung einer Hepatitis-C-Virus-Infektion.

3. Verwendung nach Anspruch 2, wobei der Hepatitis-C-Virus-NS3/4A-Protease-Inhibitor und Ritonavir in separaten Dosierungsformen vorliegen.

4. Verwendung nach Anspruch 3, wobei die separaten Dosierungsformen etwa gleichzeitig verabreicht werden.

5. Verwendung nach Anspruch 2, wobei Hepatitis-C-Virus-NS3/4A-Protease-Inhibitor und Ritonavir in einer Einzeldosierungsform vorliegen.

6. Verwendung nach einem der Ansprüche 2 bis 5, die das Verabreichen eines weiteren Mittels umfasst, das aus einem Immunmodulator, einem antiviralen Mittel, einem anderen Inhibitor der HCV-NS3/4A-Protease, einem Inhibitor eines Targets im HCV-Lebenszyklus, der keine NS3/4A-Protease ist, einem Inhibitor des inneren Ribosomeneingangs, einem Breitspektrumvirusinhibitor, einem anderen Cytochrom-P-450-Inhibitor oder Kombinationen davon ausgewählt ist.

7. Verwendung nach Anspruch 6, wobei der Immunmodulator α-, β- bzw. γ-Interferon oder Thymosin ist, das antivirale Mittel Ribavirin, Amantadin oder Telbivudin ist oder der Inhibitor eines weiteren Targets im HCV-Lebenszyklus ein Inhibitor der HCV-Helicase, -Polymerase oder -Metallprotease ist.

## Revendications

1. Composition pharmaceutique comprenant: (a) l'inhibiteur de protéase NS3/4A du virus de l'hépatite C (HCV) 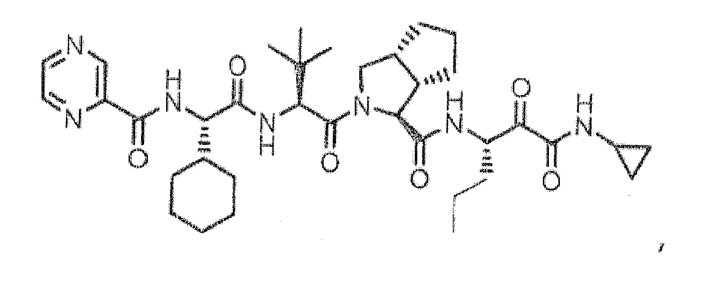 un stéréo-isomère de celui-ci ou un sel pharmaceutiquement acceptable de celui-ci; (b) du ritonavir ou un sel pharmaceutiquement acceptable de celui-ci; et (c) un support pharmaceutiquement acceptable.

2. Utilisation de l'inhibiteur de la protéase NS3/4A du virus de l'hépatite C 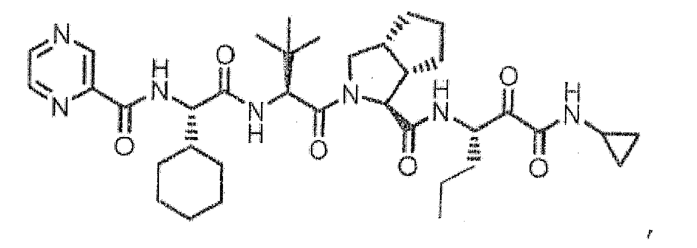 d'un stéréo-isomère de celui-ci ou d'un sel pharmaceutiquement acceptable de celui-ci et de ritonavir ou d'un sel pharmaceutiquement acceptable de celui-ci, pour la préparation d'un médicament pour traiter ou prévenir une infection par le virus de l'hépatite C.

3. Utilisation selon la revendication 2, dans laquelle l'inhibiteur de la protéase NS3/4A du virus de l'hépatite C et le ritonavir sont sous des formes pharmaceutiques différentes.

4. Utilisation selon la revendication 3, dans laquelle les formes pharmaceutiques différentes sont administrées à peu près simultanément.

5. Utilisation selon la revendication 2, dans laquelle l'inhibiteur de la protéase NS3/4A du virus de l'hépatite C et le ritonavir sont sous une forme pharmaceutique unique.

6. Utilisation selon l'une quelconque des revendications 2 à 5, dans laquelle ladite utilisation comprend l'administration d'un agent additionnel choisi parmi un agent immunomodulateur, un agent antiviral, un autre inhibiteur de la protéase NS3/4A, un inhibiteur d'une cible dans le cycle de vie du HCV autre que la protéase NS3/4A, un inhibiteur de l'entrée interne du ribosome, un inhibiteur viral à large spectre, un autre inhibiteur de cytochrome P-450, ou une combinaison de ceux-ci.

7. Utilisation selon la revendication 6, dans laquelle ledit agent immunomodulateur est une thymosine ou un interféron α, β ou γ, l'agent antiviral est la ribavirine, l'amantadine ou la telbivudine; ou l'inhibiteur d'une autre cible dans le cycle de vie du HCV est un inhibiteur de polymérase, de métalloprotéase ou d'hélicase de HCV.
